# EUROPÄISCHE PATENTSCHRIFT

(11) **EP 1 281 714 B1**
(45) Veröffentlichungstag und Bekanntmachung des Hinweises auf die Patenterteilung: **10.08.2005**
(21) Anmeldenummer: 02016299.6
(22) Anmeldetag: 06.06.1996
(51) Int. Cl.: C07H 1/08, C12N 15/10, B03C 1/01, C12Q 1/68

(54) **Magnetisches Pigment**
Magnetic pigment
Pigment magnetique

(30) Priorität: 08.06.1995 DE 19520398; 12.10.1995 DE 19537985
(43) Veröffentlichungstag der Anmeldung: 05.02.2003
(62) Teilanmeldung aus: 96921935.1
(73) Patentinhaber: Roche Diagnostics GmbH, 68305 Mannheim (DE)
(72) Erfinder: Kleiber, Jörg, 82377 Penzberg (DE); Walter, Thomas, 82377 Penzberg (DE); Harttig, Herbert, 67122 Altrip (DE); Mennig, Martin, 66287 Quierschied (DE); Lesniak, Christoph, 87474 Buchenberg (DE); Riedling, Michael, 82377 Penzberg (DE); Schmidt, Helmut K., 66130 Saarbrücken-Güdingen (DE)
(74) Vertreter: Weiss, Wolfgang, Dipl.-Chem. Dr.

(56) Entgegenhaltungen:
- EP-A- 0 343 934
- WO-A-88/06633
- WO-A-91/12079
- DE-A- 4 307 262
- GB-A- 2 116 206
- US-A- 4 554 088
- W. PYERIN ET AL.: "Filter-Supported Preparation of Phage DNA" ANAL. BIOCHEM., Bd. 175, 1988, Seiten 196-201, XP000600839
- T.L. HAWKINS ET AL.: "DNA Purification and Isolation Using a Solid-Phase" NUCLEIC ACIDS RES., Bd. 22, 1994, Seiten 4543-4, XP000602048

## Beschreibung

Gegenstand der Erfindung sind Verfahren zur Isolierung von Nukleinsäuren unter Verwendung von Partikeln mit einer Oberfläche aus siliciumhaltigen amorphem Material in Gegenwart chaotroper Salze.

Manche biologischen Materialien, insbesondere Nukleinsäuren, stellen im Hinblick auf ihre Isolierung aus der natürlichen Umgebung besondere Anforderungen. Zum einen sind sie oft in sehr geringen Konzentrationen vorhanden und zum anderen befinden sie sich oft in Nachbarschaft vieler anderer fester und gelöster Substanzen, die ihre Isolierung, beziehungsweise Bestimmung, beeinträchtigen.

In jüngerer Zeit hat es daher nicht an Versuchen gefehlt, Verfahren und Materialien zur Isolierung von Nukleinsäuren aus ihrer natürlichen Umgebung vorzuschlagen. Aus Proc. Natl. Acad. USA 76, 615 - 619 (1979) ist die Bindung von Nukleinsäuren aus Agarosegelen in Gegenwart von Natriumjodid in gemahlenem Flintglas beschrieben.

In Anal. Biochem. 121, 382 - 387 (1982) ist die Reinigung von Plasmid DNA aus Bakterien an Glasstaub in Gegenwart von Natriumperchlorat beschrieben.

In DE-A 37 34 442 ist die Isolierung von einzelsträngiger M13 Phagen-DNA an Glasfaserfiltern durch Ausfällung der Phagenpartikel mit Hilfe von Essigsäure und Lyse der Phagenpartikel mit Perchlorat beschrieben. Die an die Glasfaserfilter gebundenen Nukleinsäuren werden nach Waschen mit einem methanolhaltigen Puffer in Tris/EDTA-Puffer eluiert. In Anal. Biochem. 175, 196- 201 (1988) ist ein ähnliches Verfahren zur Reinigung von DNA aus Lambdaphagen beschrieben.

Den bisher genannten Verfahren des Standes der Technik ist die selektive Bindung von Nukleinsäuren an Glasoberflächen in chaotropen Salzlösungen gemeinsam, wobei die Nukleinsäure von Verunreinigungen wie Agarose, Proteinen oder Zelltrümmern abgetrennt wird. Zur Separierung der Glaspartikel von den Verunreinigungen wird nach dem Stand der Technik entweder Zentrifugation von Partikeln oder Durchsaugen von Flüssigkeiten durch Glasfaserfilter verwendet. Hierbei handelt es sich jedoch um einen limitierenden Schritt, der die Verarbeitung großer Probenzahlen stark behindert.

In Anal. Biochem. 201, 166 - 169 (1992) bzw. PCT GB 91/00212 ist die Verwendung von Magnetpartikeln zur Immobilisierung von Nukleinsäuren nach Ausfällung durch Zugabe von Salz und Ethanol beschrieben. Hierbei findet eine Agglutination der Nukleinsäuren unter Einschluß der Magnetpartikel statt. Das Agglutinat wird von dem ursprünglichen Lösungsmittel durch Anlegen eines Magnetfeldes und Waschen getrennt. Nach einem Waschschritt werden die Nukleinsäuren in einem Trispuffer gelöst. Dieses Verfahren hat jedoch den Nachteil, daß die Ausfällung nicht selektiv für Nukleinsäuren ist, sondern eine Vielzahl von festen und gelösten Stoffe mitagglutiniert werden. So ist es durch dieses Verfahren nicht möglich, eventuell vorhandene Inhibitoren für bestimmte enzymatische Reaktionen in ausreichendem Maße zu entfernen.

In US-A-4,233,169 ist ein poröses Glas beschrieben, das Magnetpartikel eingelagert enthält.

Auf dem Markt befindet sich derzeit auch sogenanntes magnetisches, poröses Glas, welches Magnetpartikelchen in einer porösen, partikulären Glasmatrix enthält, und wobei die Oberfläche von einer streptavidinhaltigen Schicht überzogen ist. Dieses Produkt kann zur Isolierung von biologischen Materialien, zum Beispiel Proteinen oder Nukleinsäuren verwendet werden, wenn diese in einem aufwendigen Vorbereitungsschritt so modifiziert werden, daß diese kovalent an Biotin gebunden sind.

Aufgabe der Erfindung war es, ein einfaches und für die Routine-Diagnostik geeignetes Verfahren zur Isolierung von Nukleinsäuren, bereitzustellen.

Gegenstand der Erfindung ist die Isolierung von Nukleinsäuren unter Verwendung magnetischer Partikel mit einer äußeren Glasoberfläche in Gegenwart chaotroper Salze.

Als Partikel bezeichnet der Fachmann feste Materialien mit einem geringen Durchmesser. Manchmal bezeichnet man solche Partikel auch als Pigmente. Im Sinne der vorliegenden Erfindung sind besonders Partikel geeignet, die eine durchschnittliche Korngröße von weniger als 100 µm haben. Besonders bevorzugt weisen sie eine durchschnittliche Korngröße von zwischen 10 und 60 µm auf. Bevorzugt ist die Korngrößenverteilung relativ homogen, insbesondere liegen nahezu keine Teilchen < 10µm oder > 60 µm vor.

Als magnetisch werden Materialien bezeichnet, die durch einen Magnet angezogen werden können, d. h. beispielsweise ferromagnetische oder superparamagnetische Materialien. Als magnetisch werden auch Materialien verstanden, die als weich magnetische Materialien bezeichnet werden, z. B. Ferrite. Besonders bevorzugt im Sinne der Erfindung sind ferromagnetische Materialien, insbesondere wenn sie noch nicht vormagnetisiert wurden. Unter Vormagnetisierung ist in diesem Zusammenhang das Inkontaktbringen mit einem Magneten zu verstehen, wodurch die Remanenz erhöht wird. Besonders bevorzugt werden ferromagnetische Materialien, wie z. B. Magnetit (Fe₃O₄) oder Fe₂O₃ verwendet.

Unter einer äußeren Oberfläche eines Partikels wird die zusammenhängende Oberfläche verstanden, von der in Richtung auf die Umgebung des Partikels Senkrechte gebildet werden können, die dasselbe Partikel nicht noch einmal schneiden.

Unter eine Pore wird eine Ausnehmung in der äußeren Oberfläche des Partikels verstanden, bei denen die Oberfläche soweit in das Partikel hineinreicht, daß eine in der Ausnehmung auf der Oberfläche gebildete gedachte Senkrechte in Richtung auf die nächstliegende Umgebung des Partikels das Partikel mindestens 1mal schneidet. Poren reichen außerdem tiefer als ein Radius der Pore in das Partikel hinein.

Unter einem Glas im Sinne der vorliegenden Erfindung wird ein siliciumhaltiges amorphes Material verstanden. Das Glas kann weitere Materialien enthaltene, z. B.

| | |
|---|---|
| B₂O₃ | (0 - 30 %), |
| Al₂O₃ | (0 - 20 %), |
| CaO | (0 - 20 %), |
| BaO | (0 - 10 %), |
| K₂O | (0 - 20 %), |
| Na₂O | (0 - 20 %), |
| MgO | (0 - 18 %), |
| Pb₂O₃ | (0 - 15 %). |

In geringerem Umfang von 0 - 5 % können auch eine Vielzahl anderer Oxide, wie z. B. Mn₂O₃, TiO₂, As₂O₃, Fe₂O₃, CuO, CoO usw. enthalten sein. Als besonders wirksam haben sich Oberflächen einer Zusammensetzung von Borsilikatglas, Flintglas oder Silica erwiesen. Unter dem Gesichtspunkt der Ausbeute an Nukleinsäuren besonders geeignete Borsilikatgläser haben einen Boroxidgehalt von mehr als 25 %; als besonders wertvoll wurde ein Glas der Zusammensetzung SiO₂/B₂O₃ 70/30 erkannt. Besonders im geeignet Sinne der Erfindung ist die Verwendung von Gläsern, die durch den sogenannten Gel-Solprozeß und anschließendes Trocknen und Verdichten der gebildeten Schicht gebildet werden. Dieser Prozeß ist in seinen Grundzügen bekannt und wurde z. B. in C.J. Brinker, G.W. Scherer "Sol Gel science - The physics and chemistry of Sol Gel Processing", Academic Press Inc. 1990 und Sol-Gel Optics, Processing and Applications Lisa C. Klein Ed. Kluwer Academic Publishers 1994, Seite 450 ff. sowie in DE-A-1941191, DE-A-3719339, DE-A-4117041 und DE-A-4217432 beschrieben. Er wurde allerdings bisher noch nicht für magnetische Partikel beschrieben. Daß hiermit magnetische Partikel erzeugt werden können, die bei der Isolierung von Nukleinsäuren, ganz überraschende Eigenschaften haben, war nicht zu erwarten. Im Gel-Sol-Prozeß werden Alkoxide von netzwerksbildenden Komponenten, z. B. SiO₂, B₂O₃, Al₂O₃, TiO₂, ZrO₂, GeO₂ zusammen mit Oxiden und Salzen anderer Komponenten, z. B. in alkoholischer Lösung, vorgelegt und hydrolysiert. In der Gleichung ist die Herstellung von einem Natriumboroaluminiumsilikatglas aufgeführt.

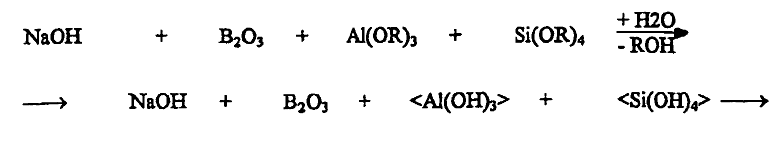

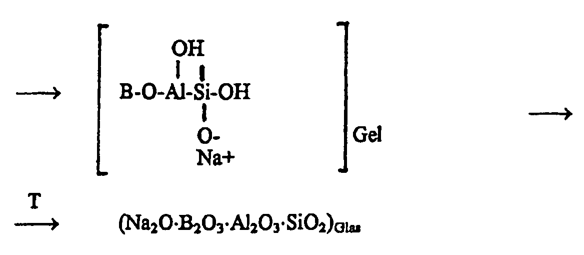

Durch die Zugabe von Wasser wird der Hydrolyseprozeß der Ausgangskomponenten in Gang gesetzt. Die Reaktion verläuft relativ rasch, da die Alkaliionen katalytisch auf die Hydrolysegeschwindigkeit des Kieselsäureesters einwirken. Nach Ablauf der Gelbildung kann das entstehende Gel getrocknet und durch einen thermischen Prozeß zu einem Glas verdichtet werden.

Das Mengenverhältnis Sol/Pigment hat einen erheblichen Einfluß auf die Ausbeute an magnetischen Pigment. Grenzen sind dadurch gegeben, daß der Pigmentanteil so gering ist, daß eine noch pump- und sprühfähige Masse entsteht. Bei zu geringem Pigmentanteil wird der Feinanteil, z. B. von nicht-magnetischem Material zu groß und stört. Als im Hinblick auf die Pigmentausbeute zweckmäßige Mengenverhältnisse wurden 10 bis 25 g Pigment/100 ml Sol gefunden.

Die Aufschlämmung wird zur Entstehung eines Pulvers bevorzugt durch eine Düse versprüht und das Aerosol auf einer Fallstrecke getrocknet. Die Düse wird bevorzugt geheizt, um die Trocknung der Aufschlämmung zu beschleunigen. Abhängig von der Geometrie der Düse beträgt die Düsentemperatur bevorzugt ca. 120 bis 200°C. Ein Kompromiß wird gefunden durch ausreichende Verdampfungsgeschwindigkeit, jedoch Vermeiden von Verspritzen.

Im Hinblick auf die Ausbeute ist die Verdichtungstemperatur möglichst hoch zu wählen. Ist sie jedoch zu hoch, verkleben die Partikel untereinander und es bilden sich Agglomerate, die herausgesiebt werden sollten. Die Nachbehandlung unter Luft führt bei zu hohen Temperaturen zu einem Verlust der magnetischen Eigenschaften, weshalb zu hohe Temperaturen vermieden werden sollten.

Für das erfindungsgemäße Verfahren können magnetische Partikel mit einer äußeren Glasoberfläche, die im wesentlichen porenfrei ist, verwendet werden (S. 3, Z. 1-2).

Unter einer im wesentlichen porenfreien Oberfläche wird eine Oberfläche verstanden, die zu weniger als 5 %, bevorzugt weniger als 2 %, besonders bevorzugt weniger als 0.1 %, mit Poren der oben stehenden Definition durchsetzt ist. Sollten Poren vorhanden sein, so haben diese bevorzugt einen Durchmesser von weniger als 10, besonders bevorzugt 1 nm.

Im Sinne der Erfindung können auch Partikel verwendet werden, die einen Kern aus mit TiO₂ beschichteten Glimmer und darauf immobilisierten Magnetitpartikeln enthalten, wobei der so gebildete Verbundstoff von der Glasschicht umschlossen ist. Sowohl der Kern als auch die Magnetitpartikel sind kristallin und nicht porös. Die Räume auf der Oberfläche des Glimmers, welche nicht von den Magnetitpartikeln besetzt ist, sind von einer dickeren Glasschicht überzogen als die Spitzen der Magnetitpartikel, so daß sich eine im wesentlichen nicht-poröse Glasoberfläche ergibt.

Die Nichtporosität der magnetischen Partikel bezieht sich nur auf die äußere Oberfläche, nicht auf das Innere des Partikels, so daß das Partikel in seinem Inneren porös sein kann, wenn die Oberfläche nur von im wesentlichen porenfreiem Glas oder einer Glasoberfläche mit Poren eines Durchmessers von weniger als 10 nm umschlossen ist.

Überraschenderweise ist das erfindungsgemäße Verfahren besonders vorteilhaft zur Isolierung von Nukleinsäuren aus Proben geeignet. Insbesondere werden lange Nukleinsäuren sehr wenig oder gar nicht zerstört, wenn sie an den magnetischen Partikeln immobilisiert werden. Das Material des Kerns ist darüber hinaus eine natürliche Ressource und somit ökologisch wenig bedenklich. Die Herstellung der Partikel ist darüber hinaus sehr wenig aufwendig und kostengünstig.

Bevorzugt ist die Verwendung ferromagnetischer Partikel mit einer Glasoberfläche. Im Stand der Technik sind superparamagnetische Partikel beschrieben. Es hat sich nun herausgestellt, daß ferromagnetische Partikel, wenn sie mit einer Glasoberfläche überzogen sind, erhebliche Vorteile bei der Isolierung von Nukleinsäuren aufweisen. Solange die ferromagnetischen Partikel noch keinem Magnetfeld ausgesetzt waren, sedimentieren sie ausschließlich unter Einfluß der Schwerkraft. Sie sind durch Schütteln einfach und schnell wieder zu suspendieren. Der Vorgang der Abscheidung ohne Magnetfeldeinfluß verläuft dabei bevorzugt langsamer als die Immobilisierung von Nukleinsäuren an ihrer Oberfläche. Die ferromagnetischen Partikel können auf einfache Weise mittels eines Magneten an einer bestimmten Stelle der Probenflüssigkeit gesammelt werden, um die Flüssigkeit von den Partikeln und somit den immobilisierten Nukleinsäuren zu trennen.

Die Glasoberfläche der ferromagnetischen Partikel kann porenfrei sein oder aber Poren enthalten. Aus den oben genannten Gründen ist es bevorzugt, daß die äußere Oberfläche auch der ferromagnetischen Partikel im wesentlichen porenfrei ist oder Poren eines Durchmessers von weniger als 10 nm aufweist. Die ferromagnetischen Partikel haben bevorzugt eine Korngröße zwischen 10 und 60 µm, besonders bevorzugt 20 und 50 µm. Besonders bevorzugt verwendet werden Partikel, bei Partikel, bei denen eventuell in der Oberfläche vorhandene Poren einen Durchmesser von weniger als 10, besonders bevorzugt 1 nm haben. Ein Beispiel für einen ferromagnetischen Partikel ist der oben genannte Verbundstoff aus Glimmer- und Magnetitpartikeln, umschlossen von einer Glasschicht.

Gegenstand der Erfindung ist ein Verfahren zur Isolierung von Nukleinsäuren
- aus einer Probe, die die Nukleinsäuren, z.B. DNA oder RNA, enthält.

Proben im Sinne der Erfindung sind beispielsweise klinische Proben, wie Blut, Serum, Mundspülflüssigkeit, Urin, Zerebralflüssigkeit, Sputum, Stuhl, Punktate und Knochenmarkproben. Die Probe kann auch aus dem Bereich der Umweltanalytik, der Lebensmittelanalytik oder der molekularbiologischen Forschung, z. B. aus Bakterienkulturen, Phagenlysaten und Produkten von Amplifikationsverfahren, z. B. PCR, stammen.

Erfindungsgemäß werden magnetische Partikel mit einem inneren Kern verwendet, auf den die äußere Glasoberfläche aufgebracht ist. Bei dem Kern kann es sich um einen Verbundstoff, jedoch auch um einfache Eisenkerne handeln. Der Kern kann auch aus einer kristallinen oder keramischen oder glasartigen Struktur bestehen, in die Eisenoxid eingelagert ist.

Mit dem geschilderten Verfahren können native Nukleinsäuren isoliert werden. Unter nativen Nukleinsäuren werden solche verstanden, der Struktur gegenüber der der natürlich vorkommenden Nukleinsäuren nicht irreversibel verändert wurde. Dies schließt jedoch nicht die Modifizierung anderer Bestandteile der Probe aus. Sollen beispielsweise Nukleinsäuren isoliert werden, so sollen auch diese in der nativen Form, d h. nicht denaturiert, geschnitten oder durch Ankoppelung reaktiver Gruppen modifiziert sein. Der Begriff native Nukleinsäuren umfaßt daher insbesondere biotinylierte Nukleinsäuren nicht. Beispiele für native Nukleinsäuren sind Phagen-DNA oder zelluläre Nukleinsäuren aus Blut.

Modifizierte biologische Materialien umfassen Materialien, die nicht in der Natur vorkommen, z. B. Nukleinsäuren, die durch Anheftung reaktiver, nachweisbarer oder zur Immobilisierung befähigenden Gruppen modifiziert sind, z. B. biotinylierte Nukleinsäuren.

In bestimmten Fällen kann die Probe ohne Vorbehandlung in das erfindungsgemäße Isolierungsverfahren eingesetzt werden. In vielen Fällen sollte die Probe jedoch durch eine geeignete Methode aufgeschlossen und die in der Probe enthaltende Nukleinsäure freigesetzt werden. Verfahren zum Aufschluß von Proben sind dem Fachmann bekannt und können chemischer, enzymatischer oder physikalischer Natur sein. Auch eine Kombination dieser Verfahren ist möglich. Beispielhaft genannt sei Lyse durch Ultraschall, Hochdruck oder durch Scherung, durch Alkali, Detergenzien oder chaotrope Salzlösungen, oder durch Einwirkung von Proteinasen oder Lipasen. Speziell im Hinblick auf die Aufschlußverfahren zum Erhalt von Nukleinsäuren wird auf Sambrook et al.: Molecular Cloning, A Laboratory Manual, 2nd Addition, Cold Spring Harbour Laboratory Press, Cold Spring Harbour, NY und Ausubel et al.: Current Protocols in Molecular Biology 1987, J. Viley and Sons, NY, verwiesen.

Die Probe kann neben den zu isolierenden Nukleinsäuren weitere Bestandteile, z. B. Zelltrümmer, Proteine, Salze und weitere nicht zu isolierende Stoffe in einer Flüssigkeit enthalten. Diese Probe, die bevorzugt die Nukleinsäuren in nativer Form enthält, wird unter Bedingungen, bei denen die Nukleinsäure an die Partikeloberfläche bindet, mit den Partikeln in Kontakt gebracht. Die Bedingungen sind prinzipiell bekannt. Sie richten sich auch nach der Art der Bindung, über die die Nukleinsäuren an die Oberfläche gebunden wird. Im Verfahren der vorliegenden Erfindung werden Nukleinsäuren in native Form direkt an die Glasoberfläche gebinden. Eine Modifizierung der Nukleinsäuren erübrigt sich dadurch. Die Bindung nativer Nukleinsäuren an Glaspartikel kann analog zu Verfahren des Standes der Technik erfolgen. Bevorzugt erfolgt sie in Gegenwart chaotroper Salze, wobei die Konzentration dieser Salze zwischen 2 und 8 mol/l beträgt, bevorzugt 4 bis 6 mol/l. Bei chaotropen Salzen handelt es sich z. B. um Natriumjodit, Natriumperchlorat, Guanidininiumthiocyanat, Guanidiniumisothiocyanat oder Guanidiniumhydrochlorit, ist jedoch nicht auf diese Verbindungen beschränkt.

Zum Inkontaktbringen der Probe mit den Partikeln wird die Probe mit den Partikeln vermischt und für eine für die Bindung ausreichende Zeit inkubiert. Die Länge der Inkubation ist dem Fachmann in der Regel aus der Behandlung mit nicht-magnetischen Partikeln bekannt, eine Optimierung ist durch Durchführung einer Bestimmung der Menge an immobilisierten Nukleinsäuren an der Oberfläche zu verschiedenen Zeitpunkten möglich. Hierbei können Inkubationszeiten zwischen 10 Sekunden und 30 Minuten zweckmäßig sein.

Je nach Größe und Art der magnetischen Partikel findet schon während der Inkubationszeit eine Separation der Partikel von der Flüssigkeit statt oder erhält sich die Suspension über längere Zeit. Wenn die Partikel eine sehr kleine Korngröße aufweisen und superparamagnetisch sind, erhält sich die Suspension über einen längeren Zeitraum. Handelt es sich um Partikel mit einer größeren Korngröße, so findet bereits während der Inkubation eine langsame Separation der Partikel von der Flüssigkeit statt. Insbesondere wenn es sich um ferromagnetische Partikel handelt, bilden sich solche Aggregate. Für den bevorzugten Fall, daß die ferromagnetischen Partikel nicht vormagnetisiert sind, ist eine besonders schonende Separation gewährleistet.

Die Immobilisierung findet bevorzugt nicht durch Ausfällung durch Erniedrigung der Löslichkeit der zu immobilisierten Nukleinsäuren statt. Stattdessen beruht die Immobilisierung auf Adsorption. Dies vermeidet weitgehend unspezifische Einschlüsse von Verunreinigungen.

Nach der Inkubation erfolgt die Abtrennung der gebundenen Nukleinsäuren von der Flüssigkeit. Dies wird allgemein durch die Separation der an die magnetischen Partikel gebundenen Nukleinsäuren mit Hilfe eines Magnetfeldes errreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden wurden, entfernt werden. Diese Entfernung hängt von der Art des Gefäßes ab, in dem die Inkubation stattgefunden hat. Geeignete Verfahrensschritte sind Abpipettieren oder Absaugen der Flüssigkeit.

Danach können die magnetischen Partikel gewünschtenfalls ein- oder mehrmal mit einer Waschlösung gereinigt werden. Die Waschlösung wird so gewählt, daß eine Ablösung der gebundenen Nukleinsäuren von der Partikeloberfläche möglichst nicht stattfindet, jedoch nicht zu isolierende Verunreinigungen möglichst gut weggewaschen werden. Dieser Waschschritt findet bevorzugt durch Inkubation der Waschlösung mit den Partikeln statt, wobei bevorzugt eine Resuspension der Partikel vorgenommen wird, z. B. durch Schütteln oder Anlegung eines nicht mit dem ersten Magnetfeld identischen Magnetfeldes. Die verunreinigte Waschlösung wird bevorzugt genauso entfernt wie die Probe in dem oben genannten Schritt zur Bindung der Nukleinsäuren.

Im Anschluß an den letzten Waschschritt kann ein kurzer Trocknungsschritt der magnetischen Partikel im Vakuum oder durch Ausdampfen (lassen) der Flüssigkeit vorgenommen werden, wobei auch eine Vorbehandlung mit Aceton möglich ist.

Die so gereinigten Nukleinsäuren können falls gewünscht, von den magnetischen Partikeln entfernt werden. Auch dieser Schritt richtet sich nach der Art der Bindung der Nukleinsäuren an die magnetischen Partikel. Bei nativen Nukleinsäuren und bei glasüberzogenen magnetischen Partikeln kann die Nukleinsäure mittels eines Elutionspuffers mit niedrigem Salzgehalt von den erfindungsgemäßen Partikeln entfernt werden. Solche Puffer sind aus DE 3724442 und Analytical Biochemistry 175, 196-201 (1988) bekannt. Als Elutionspuffer mit niedrigem Salzgehalt werden insbesondere Puffer mit einem Gehalt von weniger als 0,2 mol/l eingesetzt. In einer besonders bevorzugten Ausführungsform enthält der Elutionspuffer Tris. In einer anderen besonderen Ausführungsform handelt es sich bei dem Elutionspuffer um entmineralisiertes Wasser.

In einer weiteren Ausführungsform kann das beschriebene Reinigungs- und Isolierungsverfahren im Anschluß an eine immunomagnetische Separation von Zellen (z. B. virale Partikel oder prokariontische bzw. eukariontische Zellen) aus einer Körperflüssigkeit oder einem Gewebe erfolgen. Hierzu wird die Probe mit magnetischen Partikeln, an welche ein Antikörper gegen ein Antigen auf der Zelle immobilisiert ist, z. B. unter Schütteln inkubiert. Solche Partikel können erfindungsgemäße Partikel sein, aber auch käufliche (z. B. MACS Microbeads der Firma Miltenyi Biotec GmbH, Bergisch Gladbach, BRD). Nach Anlegen eines Magnetfeldes erfolgen ein oder mehrere Waschschritte mit einer salzhaltigen Waschlösung. Man erhält Partikel, an welche die gewünschten Zellen gebunden sind. Schließlich werden die gebundenen Zellen in einem salzhaltigen Puffer resuspendiert. In einer bevorzugten Ausführungsform ist dieser salzhaltige Puffer eine chaotrope Salzlösung, so daß die in der Zelle vorhandenen Nukleinsäuren aus den Zellen freigesetzt werden.

Durch Kombination der oben beschriebenen Isolierung von Zellen mit der ebenfalls beschriebenen Isolierung von Nukleinsäuen, bevorzugt in ihrer nativen Form, an den erfindungsgemäßen magnetischen Partikeln, ergibt sich ein besonders vorteilhaftes Verfahren zur Isolierung von Nukleinsäuren aus zellhaltigen Proben. Vorteil dieser Ausführungsform ist die mögliche Einfachheit (Single-Tube-Methode), hohe Sensitivität (besonders wichtig in der medizinischen Mikrobiologie und Onkologie) und die leichte Automatisierbarkeit.

Die als Folge der erfindungsgemäßen Verfahren isolierten Nukleinsäuren können nun in beliebiger Weise weiter verwendet werden. Beispielsweise können sie als Substrat für verschiedene enzymatische Reaktionen verwendet werden, z.B. zur Sequenzierung, zur radioaktiven oder nicht-radioaktiven Markierung, zur Amplifikation einer oder mehrerer in ihr enthaltender Sequenzen, zur Transkription, Hybridisierung mit markierten Sondennukleinsäuren, Translation oder zur Ligation. Ein Vorteil des erfindungsgemäßen Verfahrens ist, daß die Abtrennung der Nukleinsäuren von der Flüssigkeit sehr einfach ist. Im Stand der Technik wurde zur Separierung von Glaspartikeln von Verunreinigungen entweder ein Zentrifugationsschritt angewandt, oder im Falle der Bindung des biologischen Materials an Glasfiberfilter die Flüssigkeit durch diese Flüssigkeit gesaugt. Hierbei handelt es sich um einen limitierenden Schritt, der die Verarbeitung von großen Probenzahlen behindert.

Mit dem erfindungsgemäßen Verfahren ist eine effektivere Abtrennung der Nukleinsäuren von Verunreinigungen möglich. Insbesondere können Inhibitoren für bestimmte enzymatische Reaktionen erfindungsgemäß in besonders gutem Umfang entfernt werden. Die Ausbeute an Nukleinsäuren ist vergleichsweise hoch. Eine Fraktionierung langer Nukleinsäuren wurde nicht beobachtet. Verwendet werden bevorzugt Partikel, welche schneller magnetisierbar sind.

In Figur 1 ist schematisch eine Isolierung von Nukleinsäuren aus einer zellhaltigen Probe gezeigt.

In Figur 2 ist die Auftrennung erfindungsgemäß isolierter Nukleinsäuren in einem Agarosegel gezeigt.

In Figur 3 ist die Auftrennung von Reaktionsprodukten nach erfindungsgemäßer Isolierung und PCR-Amplifikation dargestellt.

In Figur 4 ist ein Gel der Ergebnisse aus Beispiel 4 gezeigt.

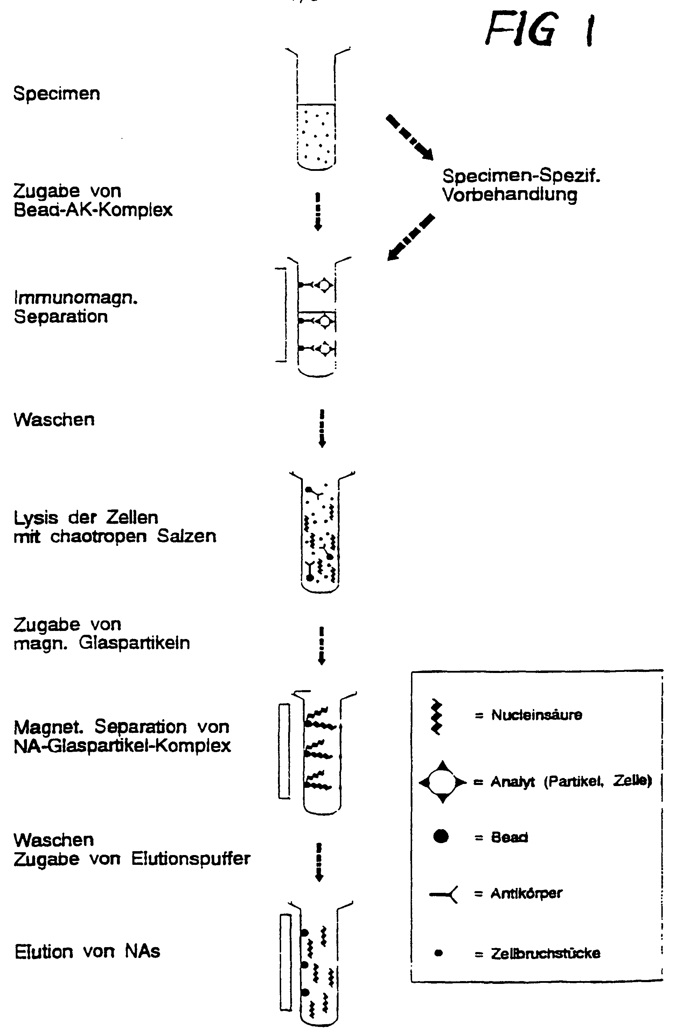

In Figur 1 ist eine Isolierung von Nukleinsäuren aus einer zellhaltigen Probe schematisch dargestellt. Die Probe (Specimen), welche Zellen enthält, wird probenspezifisch so vorbehandelt, daß die Zellen, in denen die Nukleinsäuren nachgewiesen werden sollen, in geeigneter Form vorliegen. Hierzu gehört z. B. bei Proben, welchen Körperflüssigkeiten entnommen wurden, die Zugabe von Reagenzien, z. B. zur Verflüssigung von zähflüssigen Proben, z. B. Speichelproben. Der so vorbereiteten Probe wird in einem Gefäß ein an eine Festphase, bevorzugt an eine Perle (Bead) gebundener Antikörper zugegeben, welcher die Zelle erkennen und binden kann. Als geeignete Partner für den Antikörper haben sich beispielsweise Antigene auf der Zelloberfläche erwiesen. Die Spezifität des Antikörpers kann sich nach der Spezifität der zu lösenden Analyseaufgabe richten. Wenn es sich bei der Festphase um die Wand des Gefäßes handelt, werden die Zellen direkt an die Wand gebunden. Für den Fall, daß es sich bei den Festphasen um eine Perle handelt, werden diese durch geeignete Separationsmethoden von der Flüssigkeit separiert. Dies kann beispielsweise durch Filtration geschehen. Im Falle von magnetischen Perlen ist eine Abtrennung durch Anlegen eines Magnetfeldes an die Außenwand des Gefäßes möglich. Die separierten Zellen werden mit einer Flüssigkeit gewaschen, um Verunreinigungen, welche den Nachweis stören würden, mit dem die Zellen umgebenden Medium zu entfernen. Bevorzugt werden Bedingungen eingesetzt, bei denen die Zellen weder von der Festphase gelöst noch zerstört werden. Anschließend findet die Zerstörung der Zellen statt, die sogenannte Lyse. Eine Möglichkeit ist durch die Behandlung der Zellen mit chaotropen Salzen gegeben. Andere Möglichkeiten sind die Einwirkung von Proteinasen und Detergenzien.

Zu der Lysemischung werden in der bevorzugten Ausführungsform die magnetischen Partikel/zugegeben. Nach einer geeigneten Einwirkungszeit, die über die Beladung der Oberfläche mit Nukleinsäuren optimiert werden kann, werden die Partikel von der sie umgebenden Flüssigkeit, die weitere und nicht nachzuweisende Zellbestandteile enthält, getrennt. Dies geschieht wiederum bevorzugt durch Anlegen eines magnetischen Feldes mittels eines Magneten an der Gefäßwand.

Um eventuell noch anhaftende Verunreinigungen zu entfernen, wird bevorzugt mit einer Flüssigkeit gewaschen, die so ausgewählt wird, daß die zu bestimmenden Nukleinsäuren sich nicht von der Glasoberfläche ablösen. Zur Entfernung der Nukleinsäuren von der Glasoberfläche wird ein sogenannter Elutionspuffer zugegeben, der Reagenzbedingungen aufweist, unter denen sich die Nukleinsäuren von der Glasoberfläche lösen. Dies sind insbesondere Niedrigsalzbedingungen. Je nach beabsichtigter Weiterbehandlung der Nukleinsäuren kann die Flüssigkeit nun von den Partikeln getrennt und weiterbearbeitet werden. Es ist bevorzugt, diese Abtrennung bei anliegendem Magnetfeld vorzunehmen, so daß die Partikel separiert vorliegen.

Die folgenden Beispiele erläutern die Erfindung näher.

### Beispiel 1

### Herstellung magnetischer Partikel

Es wurden 6 verschiedene Sole verwendet. Die Herstellung der Sole wurde nach folgenden Schemata durchgeführt:
Sol 1 (SiO₂:B₂O₃ = 7:3):
   Die Synthese wurde in einem 250 ml Rundkolben unter ständigem Rühren durchgeführt. 86,6 ml Tetraethylorthosilicat
   + 7 ml wasserfreies unvergälltes Ethanol
   + 14,1 ml 0,15 M HCl
   Es entsteht ein zweiphasiges Gemisch, welches bei Raumtemperatur solange gerührt wird, bis es einphasig wird. Danach erfolgt tropfenweise die Zugabe von
   + 37,8 ml Trimethylborat.
   Anschließend wird das Sol 2 Stunden bei 50 °C gehalten. Danach erfolgt die Zugabe von
   + 14,1 ml 0, 15 M HCl
Sol 2 (SiO₂:B₂O₃ = 4:1):
   Die Synthese wurde in einem 250 ml Rundkolben unter ständigem Rühren durchgeführt. 100,5 ml Tetraethylorthosilicat
   + 7 ml wasserfreies unvergälltes Ethanol
   + 16, 3 ml 0,15 M HCl
   Es entsteht ein zweiphasiges Gemisch, welches bei Raumtemperatur solange gerührt wird, bis es einphasig wird. Danach erfolgt tropfenweise die Zugabe von + 25,6 ml Trimethylborat.
   Anschließend wird das Sol 2 Stunden bei 50 °C gehalten. Danach erfolgt die Zugabe von + 16,3 ml 0,15 M HCl
Sol 3 (SiO₂:B₂O₃ = 85:15):
   Die Synthese wurde in einem 250 ml Rundkolben unter ständigem Rühren durchgeführt. 107,8 ml Tetraethylorthosilicat
   + 7 ml wasserfreies unvergälltes Ethanol
   + 17,5 ml 0,15 M HCl
   Es entsteht ein zweiphasiges Gemisch, welches bei Raumtemperatur solange gerührt wird, bis es einphasig wird. Danach erfolgt tropfenweise die Zugabe von + 19,4 ml Trimethylborat.
   Anschließend wird das Sol 2 Stunden bei 50 °C gehalten. Danach erfolgt die Zugabe von + 17,5 ml 0,15 M HCl
Sol 4 (SiO₂:B₂O₃ = 4:1; 2 Mol% P₂O₅):
   Die Synthese wurde in einem 250 ml Rundkolben unter ständigem Rühren durchgeführt. 100,5 ml Tetraethylorthosilicat
   + 7 ml wasserfreies unvergälltes Ethanol
   + 16,3 ml 0,15 M HCl
   Es entsteht ein zweiphasiges Gemisch, welches bei Raumtemperatur solange gerührt wird, bis es einphasig wird. Danach erfolgt tropfenweise die Zugabe von
   + 25, 6 ml Trimethylborat
   Anschließend wird das Sol 2 Stunden bei 50 °C gehalten. Danach erfolgt die Zugabe von + 16,3 ml 0,15 M HCl
   + 1,63 g P₂O₅
Sol 5 (SiO₂:B₂O₃ = 4:1 Mol% Al₂O₃):
   Die Synthese wurde in einem 250 ml Rundkolben unter ständigem Rührem durchgeführt. 100,5 ml Tetraethylorthosilicat
   + 7 ml wasserfreies unvergälltes Ethanol
   + 16,3 ml 0, 15 M HCl
   Es entsteht ein zweiphasiges Gemisch, welches bei Raumtemperatur solange gerührt wird, bis es einphasig wird. Danach erfolgt tropfenweise die Zugabe von
   + 25,6 ml Trimethylborat.
   Anschließend wird das Sol 2 Stunden bei 50 °C gehalten. Danach erfolgt die Zugabe von + 16,3 ml 0,15 M HCl
   + 3,06 g AlCl₃
Sol 6 (SiO₂:B₂O₃ = 4:1 Mol% ZrO₂):
   Die Synthese wurde in einem 250 ml Rundkolben unter ständigem Rühren durchgeführt. 100,5 ml Tetraethylorthosilicat
   + 7 ml wasserfreies unvergälltes Ethanol
   + 16,3 ml 0,15 M HCl
   Es entsteht ein zweiphasiges Gemisch, welches bei Raumtemperatur solange gerührt wird, bis es einphasig wird. Danach erfolgt tropfenweise die Zugabe von
   + 25,6 ml Trimethylborat
   + 5,15 ml Zirkon(IV)-proylat, 70 Gew.% Lsg in 1-Propanol
   Anschließend wird das Sol 2 Stunden bei 50 °C gehalten. Danach erfolgt die Zugabe von + 16,3 ml 0,15 M HCl

Nach weiteren 2 Stunden bei 50 °C wurde in jeweils 150 ml der Sole 22,5 g Iriodin 600 (Black Mica) eingerührt und anschließend mit einem Sprühtrockner (Büchi 190, Mini Spray Dryer) beschichtet. Die Düsentemperatur des Sprühtrockners betrug 134 °C.

Das durch den Sprühtrockenprozeß erhaltene Pulver wurde anschließend einer Temperaturbehandlung unter Stickstoffatmosphäre (90 l/h) unterzogen. Die Aufheizgeschwindigkeit betrug hierbei 1 k/min und die Haltezeit betrug 2 Stunden bei der Verdichtungstemperatur. Diese Temperatur lag bei der Beschichtung mit Sol 1 bei 750 °C, bei der Beschichtung mit Sol 2 bei 860 °C und bei den übrigen Beschichtungen bei 800 °C. Nach dem Sinterprozeß wurde der Ofen abgeschaltet und das Pulver auf Raumtemperatur abgekühlt. Eventuell entstandene Agglomerate wurden mit einem 50 µm Sieb ausgesiebt.

### Beispiel 2

### Herstellung von GMP1, GMP2, GMP3 und GMP4

GMP1, GMP2, GMP3 und GMP4 sind Pigmente aus unterschiedlichen Herstellungs-Chargen, die aus Sol 1 aus Beispiel 1 in einem Prozeß nach Beispiel 1 unter folgenden Bedingungen erhalten wurden:

| **Parameter** | **GMP 1** | **GMP 2** | **GMP 3** | **GMP 4** |
|---|---|---|---|---|
| Alterung des Sols (h) (30°C) | 36 | 36 | 36 | 36 |
| Pigmentanteil des Sols (g/100 ml) | 5 | 15 | 8 | 20 |
| Luftstrom der Düse (%) | 100 | 100 | 100 | 100 |
| Luftdruck (bar) | 6 | 6 | 6 | 3 |
| Düsentemperatur (°C) | 135 | 120 | 130 | 143 |
| Verdichtungstemperatur (°C) | 534 | 534 | 534 | 615 |
| O2-Nachbehandlung (1 Stunde) | (300°C) | (300°C) | 300°C) | (400°C) |
| Ausbeute an Pigment | niedrig | hoch | mittel | hoch |
| DNA-Ausbeute | niedrig | hoch | hoch | hoch |

### Beispiel 3

### PCR Probenvorbereitung aus humanen Vollblut mit magnetischen Glaspartikeln

### Isolierung der Nukleinsäure

Von drei Glasmagnetpartikel-Chargen (GMP2 - 4) wurden je ca. 10 mg in Eppendorf Reaktionsgefäßen vorgelegt. Die genauen Einwaagen sind in Tabelle 1 angegeben, es wurden Dreifach-Bestimmungen durchgeführt.

Zu je 200 µl aufgetautem Vollblut wurden 40 µl Proteinase K (20 mg/ml, hergestellt aus Lyophilisat) pipetiert und sofort gemischt. Anschließend wurden 200 µl Bindepuffer (6 M Guanidin-HCl, 10 mM Tris-HCl, 10 mM Harnstoff, 30 % Triton X-100, pH 4,4) zugegeben, gemischt und für 10 Minuten bei 70°C inkubiert. Nach Zugabe von 200 µl i-Propanol wurde für 10 Sekunden auf dem Vortex-Mischer gemischt, die Probe für 20 Minuten bei Raumtemperatur inkubiert und abermals für 10 Sekunden wie vor gemischt. Die Magnetseparation erfolgte für wenigstens 30 Sekunden im Magnetpartikelseparator von Boehringer Mannheim (Id.-Nr. 1 641 794). Der Überstand wurde abgenommen und wie weiter unten beschrieben analysiert.

Die Magnetpartikel wurden mit jeweils 500 µl Wasch-Puffer (20 mM NaCl, 10 mM Tris-HCl, pH 7,5 (25°C), 80 % Ethanol) durch 10 Sekunden mischen, 1 Minute Inkubation bei Raumtemperatur und 10 Sekunden mischen gewaschen und mit dem Magnetpartikelseparator an die Gefäßwand gezogen. Der Überstand wurde abgenommen und verworfen. Die Waschprozedur wurde wiederholt bis der Waschüberstand farblos war (insgesamt 4 x Waschen). Nun wurden die Nukleinsäuren 3 x mit jeweils 200 µl auf 70°C vorgewärmten Elutionspuffer (10 mM Tris-HCl, pH 8,5) durch 10 Sekunden mischen, 10 Minuten Inkubation bei Raumtemperatur und 10 Minuten mischen eluiert.

### Aufarbeitung des Überstandes

Der Überstand nach der 1. Bindung an die magnetischen Glaspartikel wurde folgendermaßen auf Gehalt an Nukleinsäuren überprüft: Der Überstand wurde in ein Filter-Tube (Boehringer Mannheim, Id.-Nr. 1744003, z.B. enthalten in *High Pure* PCR Product Purification Kit) gegeben und für 1 Minute bei 8000 rpm in einer Eppendorf Tischzentrifuge zentrifugiert. Der Durchlauf wird verworfen und das Filter-Tube 2 x mit 500 µl Wasch-Puffer gewaschen (Zentrifugation wie vor). Das Filter-Tube wird kurz trocken zentrifugiert und dann mit 2 x 200 µl auf 70°C vorgewärmten 1 x Elutionspuffer durch erneute Zentrifugation eluiert.

### Analyse der Eluate und des Probenüberstandes

50 µl der Eluate bzw. der über Filter-Tube aufgearbeiteten Überstände wurden mit 10 µl Proben-Puffer versetzt und davon 45 µl in einem 0,8 %igen Agarosegel elektrophoretisch bei 120 V für 90 Minuten aufgetrennt.

Verschieden Verdünnungen der Eluate bzw. der aufgearbeiteten Überstände wurden bei 260 und 280 nm in einem Uvikon 710 (Kontron) spektroskopisch vermessen.

Zwei 5 µl Aliquots der Eluate wurden als Doppelbestimmung durch Expand™ Long Template PCR (Boehringer Mannheim, Id.-Nr. 1681834) mit spezifischen Primern für das menschliche tPA-Gen (erwartete Produktlänge 15 kb) überprüft.

| Mix I | pro Ansatz | Mix II | pro Ansatz |
|---|---|---|---|
| dNTP, je 100 mM | 1 µl | Expand™ Puffer, 10 x | 5 µl |
| Primer 1, 200 ng/nl | 1 µl | Expand™ Polymerase | 0,75 µl |
| Primer 2, 225 ng/µl | 1 µl | H₂O, _{bidest.} | 19,25 µl |
| H₂O, _{bidest.} | 17 µl | | |
| | 20 µl | | 25 µl |

Mix I wird in ein dünnwandiges PCR-Tube vorgelegt mit 5 µl Eluat versetzt und Mix II zugegeben. Der Ansatz wird kurz gemischt und mit 30 µl Minearalöl überschichtet. Die Ansätze werden in einem Perkin-Elmer Thermocycler 9600 mit folgenden Programm amplifiziert:

| | | |
|---|---|---|
| 2 Minuten | 92°C | |
| | | |
| 10 Sekunden | 92°C | |
| 30 Sekunden | 65°C | 10 Zyklen |
| 12 Minuten | 68°C | |
| | | |
| 10 Sekunden | 92°C | |
| 30 Sekunden | 65°C | 20 Zyklen |
| 12 Minuten + 20 Sekunden pro Zyklus | 68°C | |
| | | |
| 7 Minuten | 68°C | |
| anschließend | 7°C | |

Die 50 µl PCR Ansätze wurden mit 10 µl Proben-Puffer versetzt und davon 45 µl in einem 0,8 %igen Agarosegel elektrophoretisch bei 120 V für 90 Minuten aufgetrennt.

### Ergebnisse

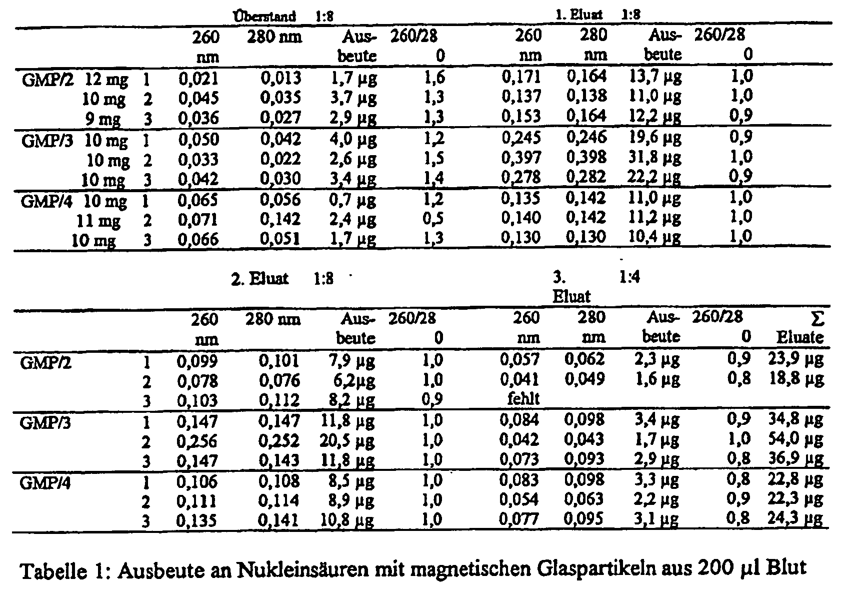

Die 1. Eluate waren noch leicht gelb gefärbt und teilweise mit feinen Magnetpartikeln kontaminiert.

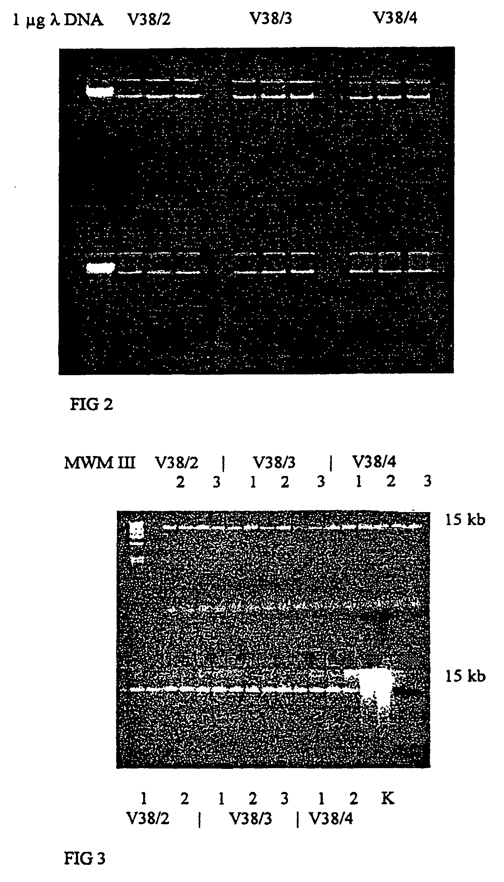

Die Analyse der Eluate im Agarosegel (FIG. 2) zeigt eine gute Reproduzierbarkeit der Ausbeute. Die Magnetpartikel GMP/2 - 4 zeigen keine signifikanten Unterschiede. In den Eluaten 1 (oben) und 2 (unten) ist etwa die gleiche Nukleinsäurekonzentration vorzufinden (vom Gel geschätzt). Eluat 3 zeigt nur noch eine geringe Nukleinsäurekonzentration. In den Überständen ist ebenfalls nur eine geringe Nukleinsäurekonzentration zu beobachten.

Die Expand™ PCR liefert mit allen Proben, bis auf wenige Ausreißer (Tabelle 2), durchweg gute und spezifische Amplifikationsprodukte. Mit den magnetischen Glasbeads ließen sich aus humanen Blutproben Nukleinsäuren isolieren, die in einer anschließenden PCR spezifische Amplifikate lieferten.

**Tabelle 2.**

| Ergebnisse Expand™ PCR | | | | | |
|---|---|---|---|---|---|
| | 15 kb Expand™ PCR | | | humanes tPA Gen | |
| | | 1. Eluat | | 2. Eluat | |
| GMP/2 | 1 | fehlt | | + | + |
| | 2 | + | + | + | + |
| | 3 | + | + | fehlt | |
| GMP/3 | 1 | + | + | + | + |
| | 2 | (+) | + | + | + |
| | 3 | - | (+) | + | + |
| GMP/4 | 1 | + | + | + | + |
| | 2 | + | + | + | (+)* |
| | 3 | + | + | fehlt | |
| K, BM Kontroll DNA | | | | | |

| | | | | | |
|---|---|---|---|---|---|
| * 3. Eluat | | | | | |

In FIG. 3 ist ein Gel mit den Reaktionsprodukten nach PCR-Amplifikation gezeigt. MWM III ist ein Molekulargewichtsmarker (Eluat 1, oben; Eluat 2, unten).

### Beispiel 4

### Bindung von DNA-Längenstandard an magnetische Glaspartikel

### 1. Vorbereitung der magnetischen Glaspartikel

Von der Glasmagnetcharge GMP4 wurden 12 mg in Eppendorf-Reaktionsgefäße vorgelegt.

### 2. Lyse und Bindung

In einem 1,5 ml Eppendorf-Gefäß mit 12 mg magnetischen Glaspartikeln werden 900 µl Lysis-Puffer (4.6 M GuSCN, 45 mM Tris, 20 mM EDTA, pH 7,3) und 100 µl DNA-Probe, in der modellhaft DNA-Längenstandard III von Boehringer Mannheim (Katalog-Nr. 528552) eingesetzt wurde, 2 bis 10 sec. gemischt, bis eine homogene Suspension entsteht. Die Lösung wird 20 min bei Raumtemperatur inkubiert, wobei alle 5 min gemischt wird.

Die Magnetseparation erfogt für mindestens 15 sec in einem Magnetpartikelseparator. Der Überstand wird abpipettiert.

### 3. Waschen und Trocknen

Die magnetischen Glaspartikel werden zweimal mit Waschpuffer (5.2 M GuSCN, 50 mM Tris, pH 6.5) zweimal mit 70% vorgekühltem Ethanol und einmal mit Aceton gewaschen, indem das Magnetfeld entfernt, 800 µl Lösung zupipettiert, 2 sec gemischt, 1 min bei RT inkubiert, das Magnetfeld angelegt und der Überstand schließlich abpipettiert wird.

Nach Entfernung des Acetons werden die Partikel 10 min bei 56 °C im Heizblock bei offenem Deckel getrocknet.

### 4. Elution der DNA

Die DNA wird mit 4x 50µl Elutionspuffer (10mM Tris-HCl, 1 mM EDTA, pH 8.0) eluiert, indem 10 min unter mehrmaligem Schütteln bei 56°C inkubiert wird und der DNA-haltige Überstand schließlich in ein neues Eppendorf-Gefäß transferiert wird.

### 5. Analyse der Eluate

Ein Fünftel des Eluatvolumens wurde mit Probenpuffer versetzt und die DNA auf einem 1 %igen Agarosegel bei 90 V aufgetrennt. Zur Bestimmung der Recovery wurde eine Verdünnungsreihe von DNA-Längenstandard III auf das gleiche Gel aufgetragen, die die in den Proben zu erwartenden DNA-Mengen enthält.

Die quantitative Auswertung erfolgte durch Scannen eines Polaroidphotos des Agarosegels. Hierbei wurde die Verdünnungsreihe des Standards als Kalibrator verwendet.

Die Ausbeute an DNA mit magnetischen Glaspartikeln ist in Tabelle 1 dargestellt

**Tabellel.**

| Ausbeute an DNA-Längenstandard III mit magnetischen Glaspartikeln | | | | | | | | |
|---|---|---|---|---|---|---|---|---|
| Standar Nr. | DNA - Menge im Stan dard [ng] | Helligkeitsintens. Standard (Meßwert) [rel.Einheiten] | Probe Nr | Pigment/ Bead-Typ | Helligkeitsintens. Probe (Meßwert) [rel.Einheiten] | errechnete DNA-Menge auf Gel [ng] | errechnete DNA-Menge in Probe [ng] | Recovery [%] |
| 1 | 200 | 65 | 1 | GMP4 | 45 | 139 | 695 | 69,5 |
| 2 | 175 | 56 | 2 | GMP4 | 39 | 120 | 600 | 60,0 |
| 3 | 150 | 51 | | | | | | |
| 4 | 125 | 44 | | | | | | |
| 5 | 100 | 37 | | | | | | |
| 6 | 75 | 25 | | | | | | |
| 7 | 50 | 17 | | | | | | |
| 8 | 25 | 9 | | | | | | |
| 9 | 10 | 4 | | | | | | |

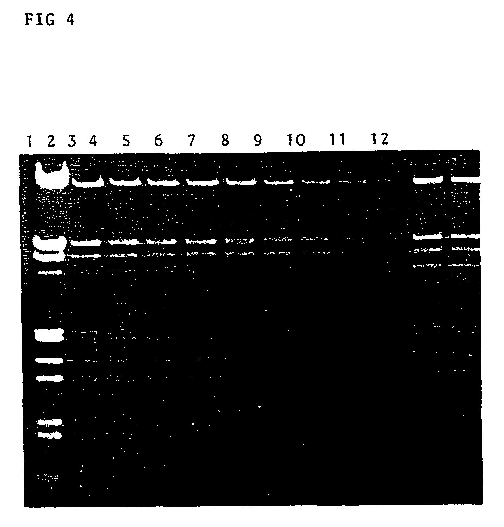

Das Agarosegel, das als Grundlage für die quantitative Auswertung diente, ist in FIG. 4 dargestellt. Es handelt sich um ein 1%iges Ethidiumbromid-gefärbtes Agarosegel. Spur 1 bis 10 entspricht einer Verdünnungsreihe von DNA-Längenstandard III. 1: 1 µg DNA, 2: 200 ng DNA, 3: 175 ng DNA, 4: 150 ng DNA, 5: 125 ng DNA, 6: 100 ng DNA, 7: 75 ng DNA, 8: 50 ng NDA, 9: 25 ng DNA, 10: 10 ng DNA.

Spur 11 und 12 entspricht der von den magnetischen Glaspartikeln eluierten DNA bei Einsatz von 200 ng DNA-Längenstandard.

## Patentansprüche

1. Verfahren zur Isolierung von Nukleinsäuren durch
- Inkontaktbringen einer Probe, welche die Nukleinsäure in einer Flüssigkeit enthält, mit magnetischen Partikeln, die eine Oberfläche aus siliciumhaltigem amorphem Material aufweisen, in Gegenwart chaotroper Salze unter Bedingungen, bei denen die Nukleinsäuren in nativer Form direkt an die Oberfläche binden können, und
- Abtrennung der gebundenen Nukleinsäuren von der Flüssigkeit.

2. Verfahren nach Anspruch 1,
**dadurch gekennzeichnet,**
**dass** die Partikel eine durchschnittliche Korngröße von weniger als 100 µm aufweisen.

3. Verfahren nach Anspruch 1 oder 2,
**dadurch gekennzeichnet,**
**dass** die Partikel einen inneren Kern aus magnetischem Material enthalten, auf den die äußere Oberfläche aufgebracht ist.

4. Verfahren nach Anspruch 3,
**dadurch gekennzeichnet,**
**dass** das magnetische Material Magnetit (Fe₃O₄) oder Fe₂O₃ ist.

5. Verfahren nach einem der Ansprüche 1 bis 4,
**dadurch gekennzeichnet,**
**dass** die magnetischen Partikel eine Glasoberfläche aufweisen.

6. Verfahren nach einem der Ansprüche 1 bis 5,
**dadurch gekennzeichnet,**
**dass** die Oberfläche der magnetischen Partikel gegebenenfalls weitere Materialien enthält.

7. Verfahren nach Anspruch 6,
**dadurch gekennzeichnet,**
**dass** die weiteren Materialien ausgewählt werden aus
| | |
|---|---|
| B₂O₃ | (0-30 %) |
| Al₂O₃ | (0-20 %) |
| CaO | (0-20 %) |
| BaO | (0-10 %) |
| K₂O | (0-20 %) |
| Na₂O | (0-20 %) |
| MgO | (0-18 %) |
| Pb₂O₃ | (0-15 %). |

8. Verfahren nach einem der Ansprüche 1 bis 7,
**dadurch gekennzeichnet,**
**dass** man eine klinische Probe, eine Probe aus dem Bereich der Umweltanalytik, der Lebensmittelanalytik oder der molekularbiologischen Forschung untersucht.

9. Verfahren nach Anspruch 8,
**dadurch gekennzeichnet,**
**dass** man eine Probe, ausgewählt aus Blut, Serum, Mundspülflüssigkeit, Urin, Zerebralflüssigkeit, Sputum, Stuhl, Punktaten und Knochenmarksproben, untersucht.

10. Verfahren nach Anspruch 8,
**dadurch gekennzeichnet,**
**dass** man eine Probe aus Bakterienkulturen, Phagenlysaten oder Produkten von Amplifikationsverfahren untersucht.

11. Verfahren nach einem der Ansprüche 1 bis 10,
**dadurch gekennzeichnet,**
**dass** man DNA isoliert.

12. Verfahren nach einem der Ansprüche 1 bis 10,
**dadurch gekennzeichnet,**
**dass** man RNA isoliert.

13. Verfahren nach einem der Ansprüche 1 bis 12,
**dadurch gekennzeichnet,**
**dass** man die Probe ohne Vorbehandlung einsetzt.

14. Verfahren nach einem der Ansprüche 1 bis 12,
**dadurch gekennzeichnet,**
**dass** man die Pobe durch eine geeignete Methode aufschließt und die darin enthaltenen Nukleinsäuren freisetzt.

15. Verfahren nach einem der Ansprüche 1 bis 14,
**dadurch gekennzeichnet,**
**dass** die Konzentration der chaotropen Salze 2-8 mol/l, vorzugsweise 4-6 mol/l beträgt.

16. Verfahren nach einem der Ansprüche 1 bis 15,
**dadurch gekennzeichnet,**
**dass** die chaotropen Salze ausgewählt werden aus Natriumjodid, Natriumperchlorat, Guanidiniumthiocyanat, Guanidiniumisothiocyanat und Guanidiniumhydrochlorid.

17. Verfahren nach einem der Ansprüche 1 bis 16,
**dadurch gekennzeichnet,**
**dass** man die Probe für eine Zeitdauer zwischen 10 Sekunden und 30 Minuten mit den Partikeln inkubiert.

18. Verfahren nach einem der Ansprüche 1 bis 17,
**dadurch gekennzeichnet,**
**dass** die Abtrennung durch Separation der an die magnetischen Partikel gebundenen Nukleinsäuren mit Hilfe eines Mangetfeldes erreicht wird.

19. Verfahren nach einem der Ansprüche 1 bis 18,
**dadurch gekennzeichnet,**
**dass** die magnetischen Partikel ein- oder mehrmals mit einer Waschlösung gereinigt werden.

20. Verfahren nach Anspruch 19,
**dadurch gekennzeichnet,**
**dass** der Waschschritt eine Inkubation der Waschlösung mit den Partikeln umfasst, wobei eine Resuspension der Partikel vorgenommen wird.

21. Verfahren nach Anspruch 20,
**dadurch gekennzeichnet,**
**dass** die Resuspension durch Anlegung eines nicht mit dem ersten Magnetfeld identischen Magnetfeldes erfolgt.

22. Verfahren nach einem der Ansprüche 19 bis 21,
**dadurch gekennzeichnet,**
**dass** nach dem letzten Waschschritt ein Trocknungsschritt erfolgt.

23. Verfahren nach einem der Ansprüche 1 bis 22,
**dadurch gekennzeichnet,**
**dass** die gereinigten Nukleinsäuren von den magnetischen Partikeln entfernt werden.

24. Verfahren nach Anspruch 23,
**dadurch gekennzeichnet,**
**dass** ein Elutionspuffer mit einem Salzgehalt von weniger als 0,2 mol/l eingesetzt wird.

25. Verfahren nach einem der Ansprüche 1 bis 24,
**dadurch gekennzeichnet,**
**dass** es als Single-Tube-Verfahren durchgeführt wird.

26. Verfahren nach einem der Ansprüche 1 bis 25,
**dadurch gekennzeichnet,**
**dass** es automatisiert durchgeführt wird.

## Claims

1. Method for isolating nucleic acids by
- contacting a sample which contains the nucleic acid in a liquid, with magnetic particles which have a surface made of a silicon-containing amorphous material, in the presence of chaotropic salts under conditions that enable the nucleic acids to directly bind in a native form to the surface and
- separating the bound nucleic acids from the liquid.

2. Method as claimed in claim 1,
**characterized in that**
the particles have an average particle size of less than 100 µm.

3. Method as claimed in claim 1 or 2,
**characterized in that**
the particles contain an inner core made of magnetic material to which the outer surface is applied.

4. Method as claimed in claim 3,
**characterized in that**
the magnetic material is magnetite (Fe₃O₄) or Fe₂O₃.

5. Method as claimed in one of the claims 1 to 4,
**characterized in that**
the magnetic particles have a glass surface.

6. Method as claimed in one of the claims 1 to 5,
**characterized in that**
the surface of the magnetic particles optionally contains other materials.

7. Method as claimed in claim 6,
**characterized in that**
the other materials are selected from
| | |
|---|---|
| B₂O₃ | (0 - 30 %) |
| Al₂O₃ | (0 - 20 %) |
| CaO | (0 - 20 %) |
| BaO | (0 - 10 %) |
| K₂O | (0 - 20 %) |
| Na₂O | (0 - 20 %) |
| MgO | (0 - 18 %) |
| Pb₂O₃ | (0 - 15 %) |

8. Method as claimed in one of the claims 1 to 7,
**characterized in that**
a clinical sample, a sample from the field of environmental analysis, food analysis or molecular biological research is examined.

9. Method as claimed in claim 8,
**characterized in that**
a sample selected from blood, serum, mouth rinse liquid, urine, cerebral fluid, sputum, stool, puncture biopsy and bone marrow samples is examined.

10. Method as claimed in claim 8,
**characterized in that**
a sample from bacterial cultures, phage lysates or products of amplification procedures is examined.

11. Method as claimed in one of the claims 1 to 10,
**characterized in that**
DNA is isolated.

12. Method as claimed in one of the claims 1 to 10,
**characterized in that**
RNA is isolated.

13. Method as claimed in one of the claims 1 to 29,
**characterized in that**
the sample is used without pre-treatment.

14. Method as claimed in one of the claims 1 to 12,
**characterized in that**
the sample is lysed by a suitable method and the nucleic acids contained therein are released.

15. Method as claimed in one of the claims 1 to 14,
**characterized in that**
the concentration of the chaotropic salts is 2 - 8 mol/l, preferably 4 - 6 mol/l.

16. Method as claimed in one of the claims 1 to 15,
**characterized in that**
the chaotropic salts are selected from sodium iodide, sodium perchlorate, guanidinium thiocyanate, guanidinium isothiocyanate and guanidinium hydrochloride.

17. Method as claimed in one of the claims 1 to 16,
**characterized in that**
the sample is incubated with the particles for a period between 10 seconds and 30 minutes.

18. Method as claimed in one of the claims 1 to 17,
**characterized in that**
the separation is carried out by separating the nucleic acids bound to the magnetic particles with the aid of a magnetic field.

19. Method as claimed in one of the claims 1 to 18,
**characterized in that**
the magnetic particles are washed once or several times with a wash solution.

20. Method as claimed in claim 19,
**characterized in that**
the washing step comprises an incubation of the wash solution with the particles during which the particles are resuspended.

21. Method as claimed in claim 20,
**characterized in that**
the resuspension is carried out by applying a magnetic field that is not identical to the first magnetic field.

22. Method as claimed in one of the claims 19 to 21,
**characterized in that**
a drying step is carried out after the last washing step.

23. Method as claimed in one of the claims 1 to 22,
**characterized in that**
the purified nucleic acids are removed from the magnetic particles.

24. Method as claimed in claim 23,
**characterized in that**
an elution buffer having a salt content of less than 0.2 mol/l is used.

25. Method as claimed in one of the claims 1 to 24,
**characterized in that**
it is carried out as a single-tube procedure.

26. Method as claimed in one of the claims 1 to 25,
**characterized in that**
it is carried out in an automated manner.

## Revendications

1. Procédé d'isolement d'acides nucléiques par
- mise en contact d'un échantillon, contenant l'acide nucléique dans un liquide, avec des particules magnétiques qui présentent une surface de matériau amorphe contenant du silicium, en présence de sels chaotropiques dans des conditions dans lesquelles les acides nucléiques sous forme native peuvent se lier directement à la surface, et
- séparation des acides nucléiques liés, du liquide.

2. Procédé selon la revendication 1, **caractérisé en ce que** les particules ont une taille de particule moyenne inférieure à 100 µm.

3. Procédé selon la revendication 1 ou 2, **caractérisé en ce que** les particules contiennent un noyau interne d'un matériau magnétique sur lequel est appliquée la surface externe.

4. Procédé selon la revendication 3, **caractérisé en ce que** le matériau magnétique est de la magnétite (Fe₃O₄) ou Fe₂O₃.

5. Procédé selon l'une des revendications 1 à 4, **caractérisé en ce que** les particules magnétiques présentent une surface de verre.

6. Procédé selon l'une des revendications 1 à 5, **caractérisé en ce que** la surface des particules magnétiques contient éventuellement d'autres matériaux.

7. Procédé selon la revendication 6, **caractérisé en ce que** les autres matériaux sont choisis parmi
| | |
|---|---|
| B₂O₃ | (0-30 %) |
| Al₂O₃ | (0-20 %) |
| CaO | (0-20 %) |
| BaO | (0-10 %) |
| K₂O | (0-20 %) |
| Na₂O | (0-20 %) |
| MgO | (0-18 %) |
| Pb₂O₃ | (0-15 %) |

8. Procédé selon l'une des revendications 1 à 7, **caractérisé en ce que** l'on examine un échantillon clinique, un échantillon du domaine de l'analyse de l'environnement, de l'analyse des aliments ou de la recherche en biologie moléculaire.

9. Procédé selon la revendication 8, **caractérisé en ce que** l'on examine un échantillon choisi parmi du sang, du sérum, un liquide de rinçage de la bouche, de l'urine, du liquide cérébral, des expectorations, des selles, des ponctions et des échantillons de moelle osseuse.

10. Procédé selon la revendication 8, **caractérisé en ce que** l'on analyse un échantillon de cultures de bactéries, de lysats de phages ou de produits de procédés d'amplification.

11. Procédé selon l'une des revendications 1 à 10, **caractérisé en ce que** l'on isole de l'ADN.

12. Procédé selon l'une des revendications 1 à 10, **caractérisé en ce que** l'on isole de l'ARN.

13. Procédé selon l'une des revendications 1 à 12, **caractérisé en ce que** l'on utilise l'échantillon sans prétraitement.

14. Procédé selon l'une des revendications 1 à 12, **caractérisé en ce que** l'on solubilise l'échantillon par une méthode appropriée et on libère les acides nucléiques qui y sont contenus.

15. Procédé selon l'une des revendications 1 à 14, **caractérisé en ce que** la concentration des sels chaotropiques est 2-8 mol/l, de préférence 4-6 mol/l.

16. Procédé selon l'une des revendications 1 à 15, **caractérisé en ce que** les sels chaotropiques sont choisis parmi l'iodure de sodium, le perchlorate de sodium, le thiocyanate de guanidinium, l'isothiocyanate de guanidinium et le chlorhydrate de guanidinium.

17. Procédé selon l'une des revendications 1 à 16, **caractérisé en ce que** l'on met l'échantillon à incuber avec les particules pendant un laps de temps entre 10 secondes et 30 minutes.

18. Procédé selon l'une des revendications 1 à 17, **caractérisé en ce que** la séparation est obtenue par séparation des acides nucléiques liés aux particules magnétiques à l'aide d'un champ magnétique.

19. Procédé selon l'une des revendications 1 à 18, **caractérisé en ce que** les particules magnétiques sont purifiées une ou plusieurs fois avec une solution de lavage.

20. Procédé selon la revendication 19, **caractérisé en ce que** l'étape de lavage comprend une incubation de la solution de lavage avec les particules, avec une remise en suspension des particules.

21. Procédé selon la revendication 20, **caractérisé en ce que** la remise en suspension s'effectue par application d'un champ magnétique différent du premier champ magnétique.

22. Procédé selon l'une des revendications 19 à 21, **caractérisé en ce que**, après la dernière étape de lavage, on effectue une étape de séchage.

23. Procédé selon l'une des revendications 1 à 22, **caractérisé en ce que** l'on sépare les acides nucléiques purifiés des particules magnétiques.

24. Procédé selon la revendication 23, **caractérisé en ce que** l'on utilise un tampon d'élution ayant une teneur en sels inférieure à 0,2 mol/l.

25. Procédé selon l'une des revendications 1 à 24, **caractérisé en ce qu'**il est effectué sous forme d'un procédé en un seul tube.

26. Procédé selon l'une des revendications 1 à 25, **caractérisé en ce qu'**il est effectué de manière automatisée.


### SEQUENZPROTOKOLL

<110> Roche Diagnostics GmbH
<120> Magnetisches Pigment
<130> 18242 EP1
<140>
   <141>
<150> DE19520398.4
   <151> 1995-06-08
<150> DE19537985.3
   <151> 1995-10-12
<150> EP96921935.1
   <151> 1996-06-06
<160> 2
<170> PatentIn Ver. 2.1
<210> 1
   <211> 34
   <212> DNA
   <213> Künstliche Sequenz
<220>
   <223> Beschreibung der künstlichen Sequenz: Oligodeoxyribonucleotide
<400> 1
   actgtgcttc ttgacccatg gcagaagcgc cttc                          34
<210> 2
   <211> 34
   <212> DNA
   <213> Künstliche Sequenz
<220>
   <223> Beschreibung der künstlichen Sequenz: Oligodeoxyribonucleotide
<400> 2
   ccttcactgt ctgcctaact ccttcgtgtg ttcc                          34